# EUROPÄISCHE PATENTSCHRIFT

(11) **EP 2 574 295 B1**
(45) Veröffentlichungstag und Bekanntmachung des Hinweises auf die Patenterteilung: **25.06.2014**
(21) Anmeldenummer: 12006665.9
(22) Anmeldetag: 22.09.2012
(51) Int. Cl.: A61B 17/50

(54) **Vorrichtung zum Entfernen von Zecken und Fremdkörpern aus der Haut von Mensch und Tier**
Device for removing ticks or other foreign objects from human or animal skin
Appareil pour enlever des tiques ou d'autres corps étrangers de la peau humaine ou animale

(30) Priorität: 27.09.2011 DE 102011114360
(43) Veröffentlichungstag der Anmeldung: 03.04.2013
(73) Patentinhaber: Dehn, Walter, 27801 Dötlingen (DE)
(72) Erfinder: Dehn, Walter, 27801 Dötlingen (DE)
(74) Vertreter: Girodo, Marco

(56) Entgegenhaltungen:
- DE-A1- 19 838 961
- DE-U1-202004 008 467
- US-A- 4 790 316
- US-A- 5 607 434
- US-B1- 6 884 240

## Beschreibung

Die Erfindung betrifft eine Vorrichtung zum Entfernen von Zecken und Fremdkörpern aus der Haut von Mensch und Tier als ein länglicher Stift.

Eine derartige Vorrichtung ist aus der US 4,790,316 bekannt, auf welcher der Oberbegriff von Anspruch 1 beruht. Weiter ist aus der DE 198 38 961 A1 eine Vorrichtung zum Entfernen von Zecken bekannt, bei der ein Plättchen mit einem sich konisch verengenden Aufnahmeschlitz vorgesehen ist.

Zeckenkarten im Scheckkartenformat sind preisgünstig herstellbar aber unhandlich in der Anwendung. An schwer zugänglichen Stellen, wie z. B. in Ohren, sind Zecken nicht erreichbar. Zeckenzangen haben den Nachteil, dass die Zecken gequetscht werden, beispielhaft sei DE 19652218 C1 für die Funktionalität genannt. Zeckenschlingen wie unter DE 199 17 570 C1 und DE 198 11 464 C1 beschrieben sind hinlänglich bekannt, diese Geräte sind sicher in der Anwendung aber sehr aufwendig in der Herstellung. Pinzetten wie unter DE 20 2006 008 680 U1 lassen sich nur mühsam zum täglichen Gebrauch mitführen. Es gibt einen so genannten Zeckenhaken EP 0 821 571 B1, der preisgünstig herstellbar ist und sich zum Entfernen von Zecken bewährt hat. Dieses Gerät hat jedoch die Nachteile, dass es zum Einen leicht verloren geht und nicht sicher in der Jackentasche zu verstauen ist. Aufgrund der mangelnden Größe ist es unhandlich und in einer Tasche kaum wieder zu finden. Der spitze Haken erhöht das ungewollte Verletzungsrisiko. DE 10 2005 006 412 A1 beschreibt eine Zeckenzange mit beidseitigen gelenkartigen Einklinkvorrichtungen, die durch Drücken funktionstüchtig wird. Das Kältesprühmittel befindet sich in einem mit Druckluft gefüllten Behälter. DE 20 2008 013 984 U1beschreibt eine Insektenentfernungsvorrichtung die durch zu betätigende Klemmelemente funktional wird. DE 10 2009 009 736 A1 beschreibt eine Vorrichtung zum Entfernen von Parasiten mit axial verschiebbar angeordneten gegeneinander beweglichen Greifzangen mit zwei Druckfedern. DE 297 07 042 U1 beschreibt eine Insektenpinzette mit einem Bedienungsknopf der den längsverschiebbaren und federbelasteten Stift veranlasst die Zangenhälften sich öffnend und hervortretend in Funktion zu setzen.

Die Erfindung soll die genannten Nachteile vermeiden, sicher und ohne Federelement funktionstüchtig sein. Die Aufgabe wird durch Anspruch 1 gelöst.

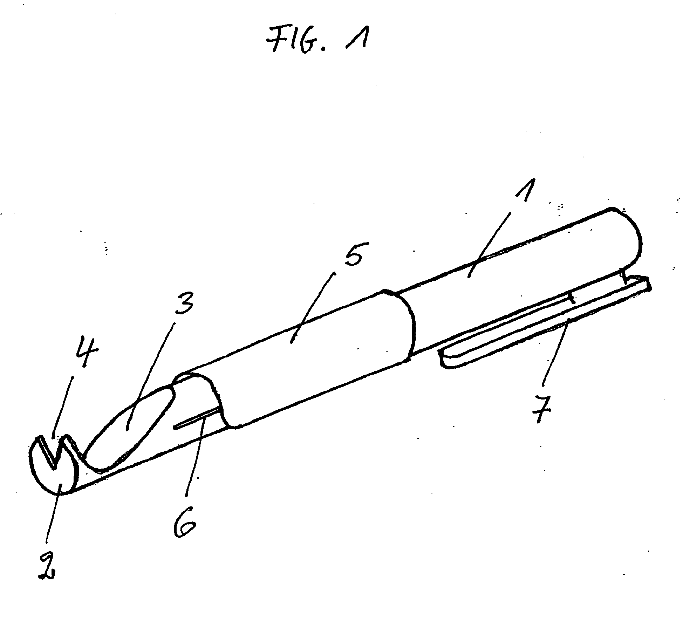

Die Vorrichtung zum Entfernen von Zecken und Fremdkörpern aus der Haut von Mensch und Tier nach Fig. 1 bietet die Vorteile der sicheren und einfachen Handhabung und der preisgünstigen Herstellung im Spritzgussverfahren.

Der Stift 1 kann problemlos durch einen Halteclip **7** in der Jackentasche getragen werden. Das Verletzungsrisiko wird durch die bewegliche Schale 5 (nachstehend Schieber genannt), der den abgeflachten, abgeschrägten distalem vorderen Ende mit dem kleinem Plättchen 2 und dem Schlitz schützt, wirksam minimiert.

Die Vorrichtung gemäß Fig. 1 ist ähnlich eines Schreibstiftes. An der "Spitze", dem vorderen Ende bzw. dem distalen Ende ist die Vorrichtung abgeschrägt 3 und an diesem Ende ist eine Platte 2 angebracht bzw. ausgebildet die im Wesentlichen senkrecht oder in einem Winkel von 40° bis 130° oder andere sinnvolle Winkel zeigt, vorzugsweise ist ein Winkel von 90° vorgesehen. Die Platte 2 weist einen sich konisch verengenden Aufnahmeschlitz 4 auf. Um die Platte 2 mit dem Aufnahmeschlitz 4 und dessen V-förmigen Rändern vor Beschädigungen zu schützen und um einer Verletzungsgefahr vorzubeugen, wird an der Vorrichtung ein beweglicher Schieber **5** in Form einer Schale angebracht, die die Vorrichtung zumindest teilweise umfasst und die in einer Position die Platte 2 mit dem Aufnahmeschlitz 4 überdeckt und in einer anderen Position den Aufnahmeschlitz 4 frei gibt.

Der Schieber 5 ist in seinem Hub nach vom zum Plättchen 2, zum proximalen Ende hin begrenzt, derart, dass ein Verlieren des Schiebers nicht möglich ist. Das wird durch mindestens eine Führungsschiene oder -nut 6 in der der Schieber 5 begrenzend beweglich ist, erreicht.

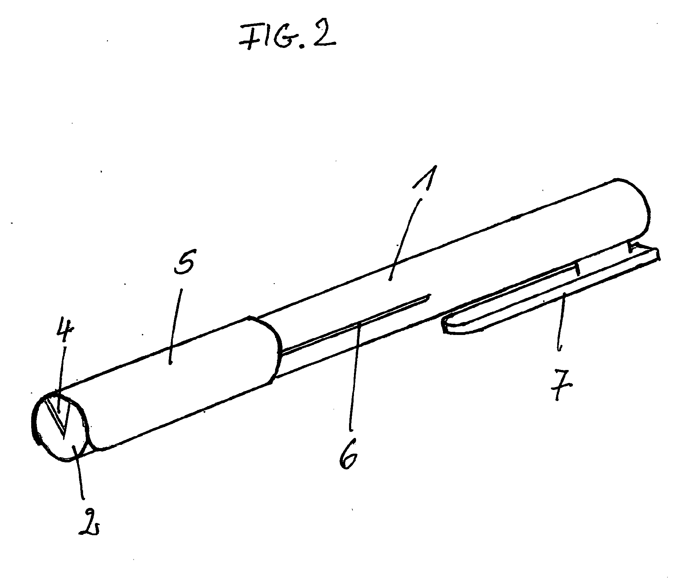

Zur Verwendung der Vorrichtung nach Fig. 1 + Fig. 2 wird die Schale 5 bzw. der Schieber 5 zurückgezogen und gibt nun das vordere Plättchen 2 mit dem konisch verlaufenden Aufnahmeschlitz 4 zur Benutzung frei. Die Vorrichtung wird an den Fremdkörper herangeführt und dieser mit dem keilförmigen Aufnahmeschlitz 4 erfasst, eingeklemmt, verkeilt und herausgezogen. Die Zecke oder der Fremdkörper wird dann von dem Plättchen entfernt, die bewegliche Schale (Schieber) 5 wird nach vom bis zum vorderen (distalen) Ende geschoben. Die Vorrichtung ist damit wieder gesichert und geschützt und kann problemlos durch den Halteclip 7 in einer Innentasche ihren Platz bis zur nächsten Verwendung finden.

Die Vorrichtung besitzt die Form eines Stiftes 1 und kann sowohl hülsenartig mit aufgesetzter Schale (Schieber) 5 gefertigt sein, wobei in diesem Fall der Halteclip 7 vorzugsweise aufgesteckt werden wird, als auch aus einen Vollmaterial bestehend gefertigt wird. Diese Vorrichtung, hier Vollmaterialstift genannt, bietet den Vorteil, dass die Vorrichtung, der Halteclip 7 und das vordere Aufnahmeplättchen 2 mit dem Keilausschnitt 4 einstückig herstellbar ist. Der schützende Schieber 5 wird im 2. Arbeitsgang aufgebracht. Die Vorrichtung kann sowohl länglich rund, abgeflacht oder mehreckig sein. Vorzugsweise ist die Vorrichtung röhrenartig rund in einer Länge von ca. 14 Zentimetern und stiftartig. Wobei die Längenangabe nicht einschränkend zu verstehen ist. So soll es etwa auch möglich sein die Vorrichtung auch in einer geringeren Länge herzustellen.

In einer weiteren, nicht beanspruchten, Ausführung ist an die Vorrichtung am vorderen, distalen Ende, in der Nähe des Plättchens 2 mit dem Aufnahmeschlitz 4, im Kegelschnitt 3 eine Lichtquelle angebracht, deren Ein- und Ausschaltfunktion über die Schale (Schieber) 5 erfolgt. Dergestalt, dass der Schieber 5 beim Zurückziehen die Lichtquelle einschaltet und anders herum wieder ausschaltet. Eine Batterie zur Versorgung der Lichtquelle wird ihren Platz innerhalb der Vorrichtung finden. Diese vorgesehene, integrierte Lichtquelle bietet den Vorteil, Fremdkörper oder Parasiten auch bei schlechten Sichtverhältnissen, z. B. im dunklen Fell eines Tieres, sichtbar zu machen.

Im unteren Teil, am distalen Ende der Vorrichtung, nahe des Plättchens 2 mit dem Aufnahmeschlitz 4 wird in einem weiteren, nicht beanspruchten, Ausführungsbeispiel eine Auslassöffnung im Kegelschnitt 3 für eine medizinisch wirksame Flüssigkeit, z. B. ein Desinfektionsmittel, über ein einfaches Membranventil vorgesehen. Im Inneren der Vorrichtung (der hülsenartige Stift) 1 befindet sich eine drucklose, leicht verformbare, Kartusche mit einem flüssigen Wirkstoff.

In DE 10 2005 006 421 A1 ist zwar eine Zeckenzange mit Sprühvorrichtung beschrieben, hier wird allerdings ein Kältespray über eine Spraydose verabreicht, so dass die Art der Applikation nicht vergleichbar ist. Vielmehr wird die hier in der beschriebenen Vorrichtung verwendete drucklose Kartusche über den niedergedrückten Halteclip in Funktion gesetzt, in dem eine Wirkverbindung zwischen Kartusche und Halteclip 7 hergestellt wird.

Alternativ ist ein Drucktaster mit Rückholfeder (nicht dargestellt) an der Vorrichtung, vorgesehen der die Möglichkeit bietet durch niederdrücken die Kartusche zu quetschen und damit den Wirkstoffaustritt gewährleistet. Somit wird die Kartusche über den niedergedrückten Halteclip oder über den Drucktaster mit einer Rückholfeder gequetscht und zur Abgabe des Wirkstoffes veranlasst.

Der vordere Teil der Vorrichtung mit dem Plättchen 2 und dem daran befindlichen keilförmigen Aufnahmeschlitz 4 als Fremdkörpererfassungsmittel kann erfindungsgemäß auch löffelartig mit Keilausschnitt oder gabelförmig, konisch zulaufend, mit zwei Zinken gestaltet sein. Wobei das Fremdkörpererfassungsmittel stets nur so groß sein soll, dass der Schieber 5 schützend zu nutzen ist. Der auf der Zeichnung Fig. 1 dargestellte flach verlaufende Kegelschnitt 3 kann erfindungsgemäß einen anderen Winkel aufweisen, solange die Funktion nicht beeinträchtigt wird. Die Vorrichtung wird zweckmäßigerweise in Kunststoff hergestellt werden. Andere Ausführungen bestehen aus Metall oder einer Zusammenführung unterschiedlicher Materialien. Die Vorrichtung muss nicht länglich rund und röhrenförmig hohl ausgebildet sein, sie kann auch erfindungsgemäß rechteckig, vieleckig, oval oder anders geformt sein.

### Bezugszeichenliste:

- 1: Stift
- 2: Plättchen am distalen Ende
- 3: Abschrägung am Stift
- 4: Keilförmiger Einschnitt im Plättchen
- 5: Bewegliche Schale/Schieber
- 6: Führungsschiene/-nut
- 7: Halteclip

## Patentansprüche

1. Vorrichtung zum Entfernen von Zecken und Fremdkörpern aus der Haut von Mensch und Tier als ein länglicher Stift (1) mit einem beweglichen Schieber (5), der die Vorrichtung zumindest teilweise umfasst und einem Plättchen (2) am distalen Ende des Stiftes (1), welches in einem Winkel von 40° bis 130° angebracht ist, **dadurch gekennzeichnet, dass** das Plättchen einen sich konisch verengenden Aufnahmeschlitz (4) mit V-förmigen Rändern aufweist und der Schieber (5) in einer Position das Plättchen (2) mit dem Aufnahmeschlitz (4) überdeckt und in einer anderen Position den Aufnahmeschlitz (4) freigibt, wobei der Schieber (5) in einer Führungsschiene oder -nut (6) des Stiftes (1) begrenzend beweglich geführt ist.

2. Vorrichtung nach Anspruch 1, **dadurch gekennzeichnet, dass** der Aufnahmeschlitz (4) des Plättchens (2) als gabelförmig, konisch zulaufend, mit zwei Zinken ausgebildet ist.

3. Vorrichtung nach Anspruch 1 oder 2, **dadurch gekennzeichnet, dass** das Plättchen (2) löffelartig mit konischem Aufnahmeschlitz gestaltet ist.

4. Vorrichtung nach einem der vorhergehenden Ansprüche, **dadurch gekennzeichnet, dass** ein Halteclip (7) zum Tragen des Stiftes (1) in einer Jacke oder Innentasche vorgesehen ist.

## Claims

1. Device for removing ticks and foreign objects from human or animal skin as an elongated shaft (1) with a movable slider (5) that at least partially surrounds the device, and a plate (2) on the distal end of the shaft (1) which is affixed at an angle of 40° to 130°, **characterised in that** the plate has a conically tapering receiving slot (4) with V-shaped edges, and the slider (5) in one position covers the plate (2) equipped with the receiving slot (4), and in another position frees the receiving slot (4), wherein the slider (5) is restrictedly movably guided in a guide rail or guide groove (6) of the shaft (1).

2. Device according to claim 1, **characterised in that** the receiving slot (4) of the plate (2) is constructed in the shape of a conically tapered fork with two tines.

3. Device according to claim 1 or 2, **characterised in that** the plate (2) is designed in the form of a spoon with a conical receiving slot.

4. Device according to one of the preceding claims, **characterised in that** a holding clip (7) is provided for carrying the shaft (1) in a jacket or inside pocket.

## Revendications

1. Dispositif à enlever des tiques et des corps étrangers de la peau de l'homme et des animaux, sous forme d'un stylet allongé (1) à un curseur mobile (5), qui embrasse le dispositif au moins en partie, et une lame (2) à l'extrémité distal dudit stylet (1), qui est disposé à un angle entre 40° et 130°, **caractérisée en ce que** ladite lame présente une fente réceptrice (4) aux bords en V, qui s'amincisse en cône, et **en ce que** ledit curseur (5), en une première position, chevauche ladite lame (2) à ladite fente réceptrice (4), et dégage, en une autre position, ladite fente réceptrice (4), audit curseur (5) étant guidé de manière mobile à limitation dans un rail de guidage ou une gorge de guidage (6) dudit stylet (1).

2. Dispositif selon la revendication 1, **caractérisé en ce que** ladite fente réceptrice (4) de ladite lame (2) présente une configuration en forme fourchée, s'amincissante en cône, à deux dents.

3. Dispositif selon la revendication 1 ou 2, **caractérisé en ce que** ladite lame (2) a une configuration en forme de cuillère, à une fente réceptrice conique.

4. Dispositif selon une quelconque des revendications précédentes, **caractérisé en ce qu'**un pince de retenue (7) est pourvu pour porter ledit stylet (1) dans une veste ou une poche intérieure.
